# EUROPEAN PATENT APPLICATION

(11) **EP 2 679 146 A1**
(43) Date of publication of application: **01.01.2014**
(21) Application number: 13174054.0
(22) Date of filing: 27.06.2013
(51) Int. Cl.: A61B 3/00

(54) **Free form custom lens design manufacturing apparatus and method**

(30) Priority: 27.06.2012 US 201261664963 P
(71) Applicant: Johnson & Johnson Vision Care, Inc., Jacksonville, FL 32256 (US)
(72) Inventor: Wildsmith, Christopher, Jacksonville, FL Florida 32256 (US)
(74) Representative: Tunstall, Christopher Stephen

(57) **Abstract**

A method, system and apparatus for providing vision correction to a patient is disclosed. The method, system and apparatus involve a) obtaining at least one bare eye data measurement, habitual lens data measurement and/or fitting lens data measurement of the patient; b) providing the at least one bare eye data measurement, the habitual lens data measurement and/or the fitting lens data measurement to a free form custom lens manufacturing system; c) producing a customized lens using the free form custom lens manufacturing system; and d) providing the custom lens to the patient.

## Description

### BACKGROUND OF THE INVENTION

### 1. Field of the Invention

The present invention relates to the field of vision correction and, more particularly, to methods and systems for providing customized vision correction, and business methods associated with providing such correction. The methods and systems employ a free form custom manufacturing process to form a custom contact lens.

### 2. Description of Related Art

A large portion of the population is ametropic; i.e., their vision is less than optimum due at least in part to refractive abnormalities of the eye. For over 100 years, practitioners ranging from lens fitters to surgeons have engaged in the business of providing vision correction to the ametropic population, as technology permitted, through spectacles, contact lenses, intra-ocular lenses (IOLs), inlays and onlays.

Many visual defects, also commonly known as aberrations, may arise from refractive abnormalities that have an adverse impact on imaging properties of an eye. Multiple orders of aberrations that may affect vision can be described using a variety of mathematical models, an example of which are Zernike coefficients. In the recommended Zernicke formulation, each Zernike coefficient gives the root-mean-square (RMS) wavefront error (in microns) contributed by the particular Zernike mode and the overall RMS error is given by the square root of the sum of the squares of the individual coefficients. A set of Zernike coefficients thus gives detailed information on the relative and absolute importance of the different aberrational defects of any particular eye for the specified conditions of measurement. In the Zernike description, first-order polynomials describe wavefront tilt (i.e., prismatic effects) and have no effect on image quality.

Second-order polynomials describe the sphero-cylindrical errors of focus which can normally be negated by optical corrections, such as spectacles or contact lenses. The third-order modes include vertical and horizontal primary coma, and the fourth-order primary spherical aberration.

While spectacles, contact lenses, and the like generally help people see better, they do not and cannot correct all refractive error. For example, although many lens products on the market today correct for some of the more commonly known low to mid order aberrations such as myopia, hyperopia, and astigmatism, they do not address higher order aberration vision correction such as coma, trefoil, and higher order sphere due to current manufacturing process limitations.

Surgical treatment such as LASIK has been successful in addressing correction of some high order aberrations. However, such treatment may put the patient at risk and often can lead to other visual limitations such as glare, reduced nighttime vision, dry eye and myopia.

Technologies are being developed and refined in an attempt to address the aforementioned issues. For example, U.S. Patent No. 6,499,843 to Bausch & Lomb Incorporated discloses a method for providing vision correction to a patient that includes engaging the patient in a practitioner's facility; obtaining a wavefront aberration measurement of an eye of the patient; providing the wavefront aberration measurement to a custom lens supply platform in a form suitable for input to the custom lens supply platform; producing a customized lens via the custom lens supply platform according to data associated with the wavefront aberration measurement; and providing the custom lens to the patient or the practitioner. U.S. Patent No. 6,499,843 discloses that the custom lens supply platform provides for manufacturing the appropriate custom lens preferably, but not necessarily, at a location remote from the practitioner's facility by the then known manufacturing methods, including, for example, a lens laser ablation system, a lens lathing system, and a lens cast-molding system.

Laser ablation works by directing the output of a high-power laser, by computer, at the material to be cut. The material then either melts, bums, vaporizes away, or is blown away by a jet of gas. Lenses derived from laser ablation are limited in that an increase in the cuts that have to be made to obtain a desired shape results in a decrease in the optical quality of the lens.

In direct lathing, the back curve and the front surface of a lens button are machined. Lathing is time consuming and lenses derived from lathing are limited to the scope of the surface available.

Cast molding utilizes front and back curve molds that are either directly machined or injection molded. The front curve and back curve mold parts are brought together to shape the lens according to desired lens parameters. A liquid lens formulation contained within the mold is cured, for example by exposure to heat and light, to form a lens. Following cure, the mold parts are separated and the lens is removed from the mold parts. Lenses derived from cast-molding are limited to the design of the mold utilized.

U.S. Patent No. 7,905,594 to Johnson & Johnson Vision Care, Inc. discloses a method and an apparatus for forming ophthalmic lenses. The method includes positioning a substrate having an optical quality area in contact with a liquid lens formulation; transmitting actinic radiation through the substrate to polymerize a portion of the liquid lens formulation; and forming the lens, wherein the lens has a first surface portion along the optical quality area and a second surface portion that is free-formed.

All patent publications and references cited in this disclosure are incorporated by reference in their entirety herein.

Methods and systems that provide customized vision correction are desired. Preferably, such methods and systems allow patients to identify one or more contact lens products that provide more tailored vision correction than currently available contact lens products. Such methods and systems should be simple to understand and use, yet provide for patient preference and/or needs matching. The methods and systems of the present invention are particularly well suited to assist the selection of contact lens products particularly suited to provide a given patient optimum vision correction.

### SUMMARY OF THE INVENTION

Methods and systems that addresses the issues and concerns identified above are set forth by the present invention.

The term "vision correction" as used in the description of the invention refers both to a measured improvement in vision over that provided by conventional refractive correction and to the subjective evaluation of "seeing better" by the patient. The term "practitioner" or "eye care practitioner" as used herein refers appropriately to anyone qualified to fit, prescribe, or dispense vision correction devices such as spectacles, contact lenses and the like, or medically attend to a patient particularly with respect to the patient's eyes.

In accordance with an aspect of the invention, a method for providing vision correction to a patient is disclosed. The method comprises:
a) obtaining at least one bare eye data measurement of the patient;
b) providing the at least one bare eye data measurement to a free form custom lens manufacturing system;
c) producing a customized lens using the free form custom lens manufacturing system; and
d) providing the custom lens to the patient.

In accordance with another aspect, a method for providing vision correction to a patient is disclosed. The method comprises:
a) obtaining at least one habitual lens data measurement of the patient;
b) providing the at least one habitual lens data measurement to a free form custom lens manufacturing system;
c) producing a customized lens using the free form custom lens manufacturing system; and
d) providing the custom lens to the patient.

In accordance with yet another aspect, a method for providing vision correction to a patient is disclosed. The method comprises:
a) obtaining at least one fitting lens data measurement of the patient;
b) providing the at least one fitting lens data measurement to a free form custom lens manufacturing system;
c) producing a customized lens using the free form custom lens manufacturing system; and
d) providing the custom lens to the patient.

The custom lens may be a contact lens, an inlay, an onlay, or an IOL. The free form custom lens manufacturing system may provide for manufacture of the appropriate custom lens at a remote manufacturing facility, on-site at a retail store, at a kiosk, in a vehicle equipped with appropriate equipment, at an eye care practitioners' office, etc.

Moreover, the bare eye data measurement, the habitual lens data measurement and the fitting lens data measurement may each be obtained by suitable means known to those skilled in the art for obtaining bare eye data measurements, habitual lens data measurements and/or fitting lens data measurements. The bare eye data measurements, the habitual lens data measurements and/or the fitting lens data measurements may be obtained from the patient in the practitioner's facility; the bare eye data measurements, the habitual lens data measurements and/or the fitting lens data measurements may be transmitted to a free form custom lens manufacturing system; and a custom lens may be manufactured by the free form custom lens manufacturing system. Practice of the invention will provide the patient with vision correction and resulting visual performance from the custom lens that is better than that which would be provided to the patient using conventional means.

Another aspect of the invention provides a method for vision correction including engaging the patient in a practitioner's facility; obtaining a bare eye data measurement and/or a habitual lens data measurement and/or a fitting lens data measurement of the patient's eye; and providing a display of either a picture, a computer simulation or a graphic display. Preferably, the display is in a form that allows the patient to make a subjective evaluation of the product options which will lead to the subjective evaluation of better vision. A related aspect involves transmitting the bare eye data measurement, the habitual lens data measurement and/or the fitting lens data measurement to a free form custom lens manufacturing system in a form readable by the free form custom lens manufacturing system for producing a custom lens.

Obtaining the bare eye data measurement, the habitual lens data measurement and/or the fitting lens data measurement and patient information and presenting the simulated display of custom lens options to the patient may be accomplished automatically outside of a practitioner's facility, in similar fashion, for example, to obtaining blood pressure readings from devices located in supermarkets, at the workplace, at home, etc. The desired information could then be transmitted automatically to a practitioner (e.g., for diagnostic purposes) or to a free form custom lens manufacturing system for making lenses for the patient if desired.

A method for providing vision correction to a patient may involve measuring an ocular characteristic of the patient's eye, either by a practitioner in the practitioner's facility or remotely without practitioner intervention. The measurement includes topography data and/or wavefront aberration data. This measured data is evaluated and the evaluation produces an option matrix that compares, among other things, prospective vision correction as a function of a prospective eye treatment, cost of treatment options, etc. Based upon the evaluation, the patient can select his/her treatment option, and lens manufacturing can occur automatically upon selection.

It will be appreciated by those skilled in the art that any data transmission referred to above could be in the form of telecom or datacom, and could be sent via wire-based (optical fiber, cable, etc.) or wireless services. A preferable interface would be Internet based.

Other features and advantages of the present invention will be apparent from the detailed description of the invention and from the claims.

### BRIEF DESCRIPTION OF THE DRAWINGS

The accompanying drawings, which are incorporated in and constitute a part of this specification, illustrate aspects of the present invention and, together with the description, serve to explain the objects, advantages and principles of the invention.
Figure 1A is a block diagram showing a system and method for providing a custom lens to a patient according to the invention, wherein user/patient interactive lens criteria selection is employed;
Figure 1B is a continuation of Figure 1A;
Figure 2 is a block diagram showing a system and method for providing a custom lens to a patient according to the invention, wherein a lens on eye simulation is employed to assist with product choice confirmation;
Figure 3 is a block diagram showing a system and method for providing a custom lens to a patient according to the invention, wherein a trial lens fit is employed to assist with product choice confirmation;
Figure 4 is a block diagram showing a system and method for providing a custom lens to a patient according to the invention, wherein a lens is manufactured;
Figure 5 is a representative display of a simulation of vision correction for a given patient;
Figure 6 is a block diagram showing a system and method for providing a custom lens to a patient according to the invention;
Figure 7 is a block diagram showing a system and method for providing a custom lens to a patient according to the invention;
Figure 8 is an exemplary apparatus that may be useful in manufacturing a custom contact lens in accordance with the invention.

### DETAILED DESCRIPTION OF THE PREFERRED EMBODIMENTS

### DEFINITIONS

As used herein, "bare eye data" means the data and information taken of a patient's eye when the patient is not using any vision correction devices. A series of exams can be performed to collect bare eye data, including, e.g., a physiology exam, a topographical exam, a wavefront exam, and a refraction exam.

As used herein, "custom product" means a product having one or more parameters that are not ordinarily available in products employing standard settings.

As used herein, "DMD" or "digital micromirror device" means a bistable spatial light modulator consisting of an array of movable micromirrors functionally mounted over a CMOS SRAM. Each mirror is independently controlled by loading data into the memory cell below the mirror to steer reflected light, spatially mapping a pixel of video data to a pixel on a display. The data electrostatically controls the mirror's tilt angle in a binary fashion, where the mirror states are either +X degrees (on) or -X degrees (off). For current devices, X can be either 10 degrees or 12 degrees (nominal). Light reflected by the on mirrors then is passed through a projection lens and onto a screen. Light is reflected off to create a dark field, and defines the black-level floor for the image. Images are created by gray-scale modulation between on and off levels at a rate fast enough to be integrated by the observer.

As used herein, "DMD script" means a control protocol for a spatial light modulator and also to the control signals of any system component, such as, for example, a light source or filter wheel either of which may include a series of command sequences in time.

As used herein, "DMD start show" means a collection of time based instructional data points that may be used to control activation of mirrors on a DMD to enable a lens to be fabricated. DMD start shows may contain data associated with a regularly or irregularly spaced grid.

As used herein, "fiducial marks" are marks employed for reference or comparison. Fiducial marks enable the determination of orientation, rotation, centration and movement of a lens. Details regarding contact lenses with fiducial marks can be found in U.S. Published Application No. 20110025979.

As used herein, "fiducialized lens" means a lens that contains one or more features that assist with determining orientation, rotation, centration, and movement of the lens. A fiducialized lens may contain fiducial marks on the lens. It may also be a lens with edge features that are detectable optically, for example, a non round lens, or a lens with notches in the edge.

As used herein, "fitting lens" means a standard, preferably stabilized contact lens that is designed to aid a manufacturer in designing a custom lens. The fitting lens may have stability and measuring points incorporated in the lens to assist with measuring the rotational position of the lens and the decentration of the eye in relation to the patients eye.

As used herein, "fitting lens data" means the data and information taken of a patient's eye when the patient is using a fitting lens vision correction device. A series of exams can be performed to collect fitting lens data, including, e.g., a physiology exam, a topographical exam, a wavefront exam, and a refraction exam.

As used herein, "fixing radiation" means actinic radiation sufficient to one or more of: polymerize and crosslink essentially all fluent lens reactive media.

As used herein, "free form" means surface that is formed by crosslinking of a lens reactive mixture and is not shaped according to a cast mold.

As used herein, "habitual lens" means a lens worn by the patient on a regular basis, e.g., daily.

As used herein, "habitual lens data" means the data and information taken of a patient's eye when the patient is using a habitual lens vision correction device. A series of exams can be performed to collect habitual lens data, including, e.g., a physiology exam, a topographical exam, a wavefront exam, and a refraction exam.

As used herein, "hardware-based" means interaction to reach information contained, formulated, and delivered entirely via non-electric or electronic means. Hardware can be static (i.e., a flow chart or matrix chart), or dynamic (i.e., one or more dials, slides, drums, or the like, moved into a particular arrangement or place so as to produce one or more instructions or selections for the user).

As used herein, "fluent lens reactive media" means a reactive mixture that is flowable in either its native form, reacted form, or partially reacted form and is formed upon further processing into a part of an ophthalmic lens.

As used herein, "mechanical choices" means those choices that are visible or tangible. Mechanical choices may include base curve, diameter, center thickness, and stabilization profiles.

As used herein, "metrology" includes both theoretical and practical aspects of measurement and "metrology equipment" includes equipment capable of measuring optical and material characteristics of materials.

As used herein, "optical choices" means those choices most beneficial to improving a patient's vision. Optical choices may include low order optical aberration correction (e.g., 2nd order, 3rd order), custom low to mid order optical aberration correction (e.g., 4th order, 5th order), and custom mid to high order optical aberration correction (e.g., 6th order, 7th order).

As used herein, "physiology exam" means an exam that observes the physical appearance of the eye. Physiology exam includes, but is not limited to, a glaucoma test (e.g., tonometry test, ophthalmoscopy, optic nerve computer imaging techniques, etc.), a retinal exam (e.g., ophthalmoscopy, papillary dilation test, optomap retinal exam, etc.), checking for ulcers, a tear production test to check for dry eye syndrome (e.g., Schirmer test), checking for eye infections, etc.

As used herein, "refraction exam" means an exam wherein a patient's vision is refracted using a device that contains hundreds of combinations of lenses to determine any possible refractive error such as nearsightedness, farsightedness, astigmatism, or presbyopia. An over-refraction exam is where a similar exam is taken but with the patient wearing a contact lens.

As used herein, "software-based" means interaction to reach information contained, formulated, and delivered with devices in which one or more are electric or electronic in construction and require software code for operation. The software can be locally installed into one or more devices or remotely located.

As used herein, "store-based" means an interaction between the patient and information utilizing devices or information source elements occurring at the point of purchase (e.g., practitioner's office, pharmacy, retail store, on-line, kiosk, mobile van, etc.).

As used herein, "topographical exam" means an exam that looks at the surface features of an eye. Topographical exam includes, but is not limited to, curvature of a cornea and surface of a retina, which may help in determining certain characteristics such as: base curve measurement of a patient's eye, limbal measurements, pupil size, line of sight measurement, pupil center measurement, geometric center measurement, etc.

As used herein, "wavefront exam" means an exam that looks at the way that the light travels in an eye. A wavefront exam, which may be performed with an aberrometer, creates an optical aberration map, which is sometimes called an "optical fingerprint", and identifies optical aberrations or distortions of a patient's eye (e.g., low order, medium order, high order, Zernike, other functions or descriptors, etc.). Examples of low order optical aberrations include nearsightedness, farsightedness, and astigmatism. Examples of high order optical aberrations include coma, trefoil, and spherical aberration.

As used herein, "web-based" means an interaction between a practitioner and/or a patient and information based on communication, either in near real time or by delayed transmission, between two points, in which this connection uses in part the Internet, commonly referred to as the World-Wide-Web, where a practitioner and/or a patient is at one of the points. The practitioner and/or the patient located point can be a store or non-store location (i.e., home or office) for such a web-based interaction.

Certain information regarding a patient may be used to assist the patient in the selection of a contact lens product. The method of the present invention centers on the ability to properly identify the needs and preferences of a patient and to match these needs and preferences with an appropriate contact lens product.

Specifically, certain pieces of information regarding the patient can be used to assist the patient in the selection of contact lens products that is/are particularly adapted to satisfy the patient's needs and preferences. The methods and systems of the invention can increase the acceptance of such products by patients compared to self selection without use of such methods and systems.

In accordance with the invention, a free form custom lens manufacturing system where a custom lens based upon the bare eye data, habitual lens data and/or fitting lens data can be made and packaged for delivery to the practitioner or the patient is provided. Diagnostic equipment may be designed to automatically output the appropriate information in suitable form for the free form custom lens manufacturing system. The patient data is such that it can be converted into data that can be used to create a desired lens profile. The information can be transmitted via the Internet, however, any supporting transmission mode and transmission medium can be used. For example, the information can be transmitted by, e.g., computer, mail, wireless device, telephone, or any other modes imaginable that may be used for data transmission. Manufacture can take place at the practitioner's office or remotely. Lens design and DMD script creation can also take place at the practitioner's office or remotely.

The free form custom lens manufacturing system is suitably equipped to produce an appropriate custom lens. Accordingly, a custom contact lens, a custom inlay, a custom onlay, or a custom IOL can be made. A specific quantity of custom lenses, e.g., contact lenses, may be produced for the patient so as to be used over an extended period of time. The lenses will preferably be packaged in a customized manner for the patient. The package can then be delivered to the patient or practitioner as appropriate.

The patient may be shown, to the extent possible, what their improved vision could be as provided by one or more custom lenses as compared to no vision correction and/or currently available lenses.

Vision diagnostic information, including wavefront measurement data, personal history, practitioner information and whatever other information may be useful for constructing or maintaining one or more databases for future use is collected. The vision diagnostic information is sent via the internet to a service platform that illustratively includes an information storage server, a lens design interface, and a lens manufacturing interface. Lens design and manufacturing information is sent to and received by a custom lens platform Custom lens information is used by lens platform to produce custom packaged lens products for the patient which can be shipped to the patient at home, for example, or to the practitioner's office for fitting and/or delivery to the patient. Certain ocular characteristics of the patient will be measured, preferably wavefront aberration and possibly, in conjunction therewith, topography measurements will be obtained. The evaluation may be in the form of an option matrix so to speak wherein information about prospective vision correction and cost, among other things, can be compared against different types of eye treatment or no treatment at all. An eye treatment option can then be selected by the patient and/or the practitioner, and upon selection, the appropriate information such as wavefront aberration data, for example, can be automatically sent to the appropriate platform (in the illustrated case, a free form custom lens manufacturing system for processing.

The lens may be formed in accordance with the method disclosed in U.S. Patent No. 7,905,594. In accordance with this method, patient's lens parameters are input into a computer or other processor; the computer or other processor executes an algorithmic calculation to correlate the lens parameters to a lens to be produced; a DMD script based on the parameters is communicated to a DMD; the DMD controls transmission of actinic radiation to a reactive mixture; and a portion of the volume of the reaction mixture is polymerized to form the lens.

The patient may situate himself in a practitioner's office. At the office, a diagnostic lens is selected and fitted to the eye. The diagnostic lens will be of similar design to that which will eventually be prescribed as the custom contact lens.

A patient may engage herself with a diagnostic platform including a bare eye data measuring device, without initial practitioner intervention, that is located as a stand-alone platform. The device would be equipped with telecom or datacom capability to accept input and transmit output regarding patient data, ordering data, etc. to an appropriate respective platform. In addition, the diagnostic platform would be capable of providing the bare eye data aberration and, preferably, correction information in a display format suitable for subjective evaluation by the patient. If so desired, the patient could direct the diagnostic platform to transmit the information to a practitioner and/or to a free form custom contact lens manufacturing system where a supply of custom contact lenses could be manufactured and packaged, and shipped to the patient or the patient's practitioner.

### Examples

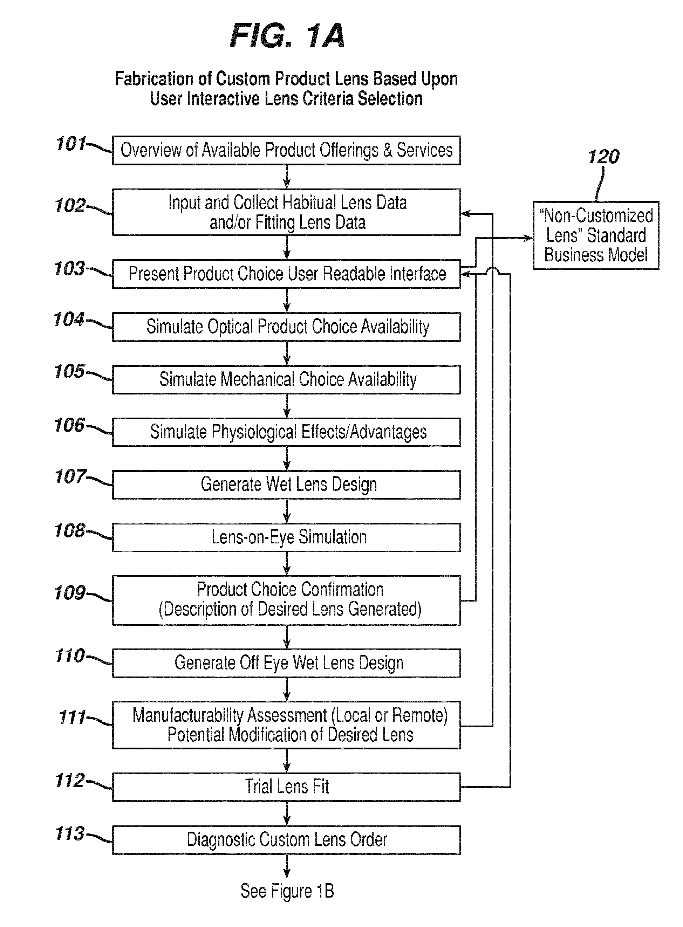

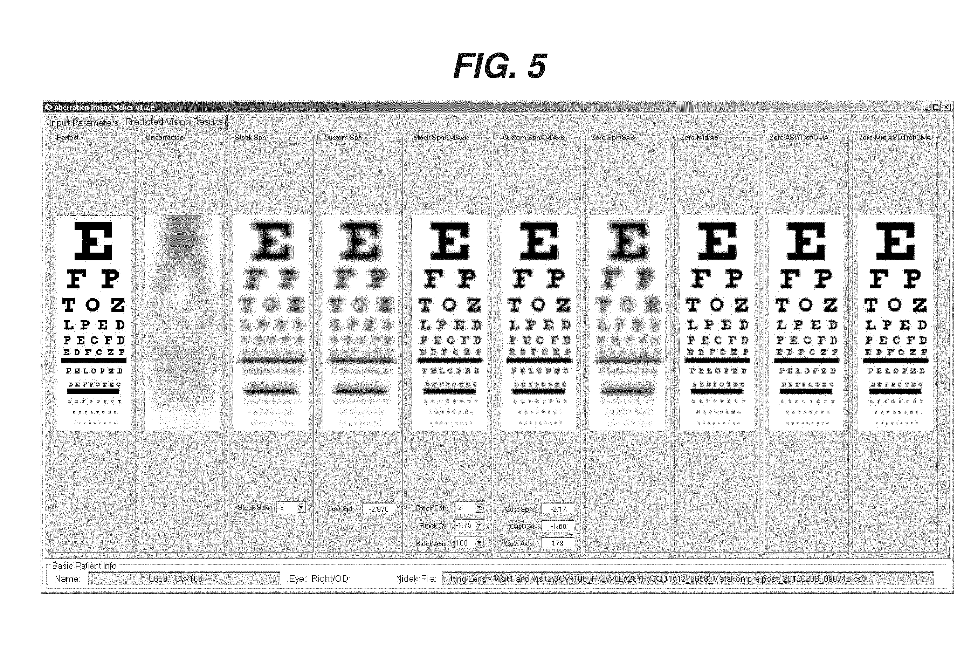

Figure 1A is a block diagram showing a system and method for providing a custom lens to a patient according to the invention, wherein user/patient interactive lens criteria selection is employed. Although the implementation of the method steps may begin at an eye care practitioner's office or at some sort of vision center, the invention should not be limited to these locations. Referring to Figure 1A, a patient is provided an overview of available products and services 101. User interactive tools that demonstrate various potential use experiences can be employed. User interactive tools can be employed to collect and store data regarding the patient 102. This data can be used to generate information regarding products and services specific to the patient 103. Such data can include the patient's age, medical conditions that the patient suffers, e.g., diabetes, hypertension, etc., eye or vision related medical conditions, e.g., glaucoma, dry-eye syndrome, etc., vision preferences, e.g., improved night vision, improved close-up vision, improved distance vision, etc.; data resulting from an eye exam, i.e., bare eye data. This data can be stored temporarily or permanently and can be employed when recommending product choices for the patient. The patient may choose to employ products and/or services currently available 120 or may choose to explore custom lens design 104 et seq. If the patient chooses to explore custom lens design 104 et seq., a simulation of optical choices 104, a simulation of mechanical choices 105 and a simulation of physiological choices 106 can be provided to the patient. Optical choices 104 can be shown using, e.g., graphical visual charts (e.g., Snellen Eye Chart), contrast sensitivity charts or by simulation of various levels of customization. For example, simulation software may be used to show a graphical version of a Snellen Eye Chart ("E" Chart) (see Figure 5) to compare a patient's bare eye vision with their corrected vision using currently available products or using one or more custom lens products. These options may include one or more of the following: custom low order optical aberration correction (e.g., 2nd order, 3rd order), custom low to mid order optical aberration correction (e.g., 4th order, 5th order), and custom mid to high order optical aberration correction (e.g., 6th order, 7th order). Mechanical choices 105 can be shown using centration/rotation models to demonstrate how a selection of certain customized mechanical options may enhance or optimize a patient's vision. Physiological choices 106 can be shown using, e.g., simulated oxygen transmission, corneal staining, and conjunctival staining by way of pressure profile graphs, surface area coverage, and contact graphs, etc. Once the patient has made optical, mechanical and physiological choices, the patient's data is used to generate a wet lens design 107 to determine how a custom lens may perform when it is on the eye. For example, simulation software may be used to display an expected performance of a patient's custom lens. This may be done by one or more of the following: displaying centration and rotation performance, displaying movement performance by possibly using a "pushup" test, and redisplaying optical performance including, either one or both of: centration and rotation performance potentially using graphical visual acuity or contrast sensitivity charts, and potentially using software such as: Zemax, Oslo, or Code V. Based on the wet lens design, a lens on eye simulation 108 is performed and the patient chooses a custom lens design 109. In cases where there may be no modification necessary to a wet lens design, data of a wet lens design may be temporarily or permanently stored.

In cases where it is determined that modification of the wet lens design may be necessary, an off eye wet lens design may be generated 110 and a manufacturability assessment may be conducted 111. A new design may be wrapped to an eye and a simulation of on-eye performance may be shown to the patient and practitioner. In cases where the modified design simulated performance is acceptable to the patient, at 103, the patient and the practitioner can revisit a product choice method step. In cases where a modified design simulated performance is acceptable to the patient and practitioner, modified design data may be temporarily or permanently stored.

A trial lens fit 112 may be performed to determine, e.g., one or more of the following: centration, rotation, and movement of the trial lens on the patient's eye 112. The trial lens fit may be done, e.g., with one or more of the following: a fiducialized lens, a non-fiducialized lens, a currently available product and a custom product. An order for a diagnostic custom lens may then be placed 113.

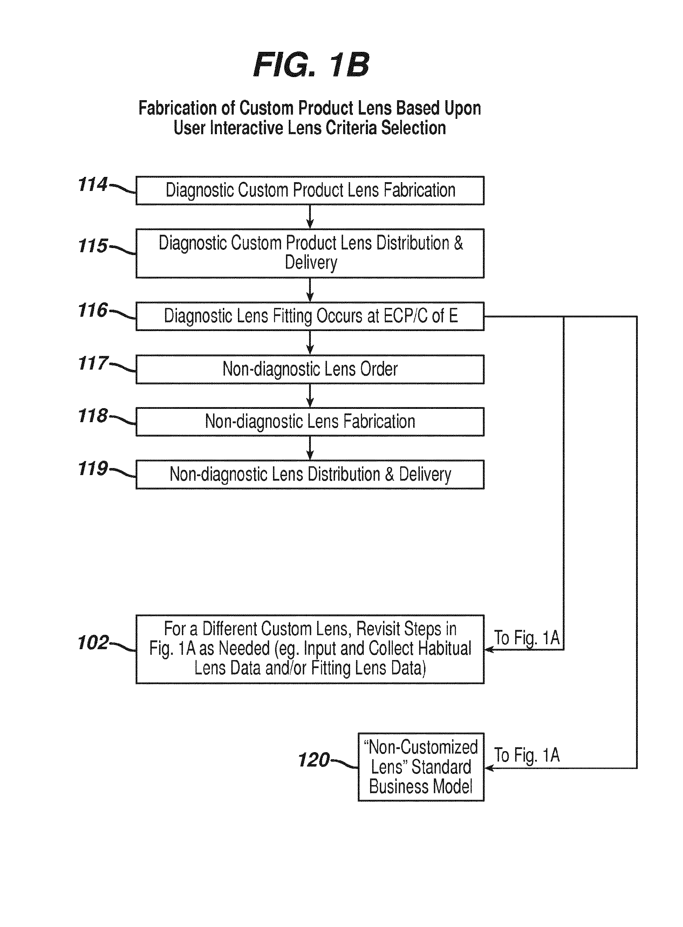

Referring to Figure 1B, which is a continuation of Figure 1A, a diagnostic custom lens is manufactured 114 and delivered to the practitioner 115. The diagnostic custom lens is fitted on the patient 116. If the diagnostic custom lens is a good fit, the final custom lens order is placed 117, the final custom lens is manufactured 118, and the final custom lens is delivered 119. If the diagnostic custom lens is not a good fit, steps 102 et seq. above can be repeated or the patient can choose to employ products and/or services currently available 120.

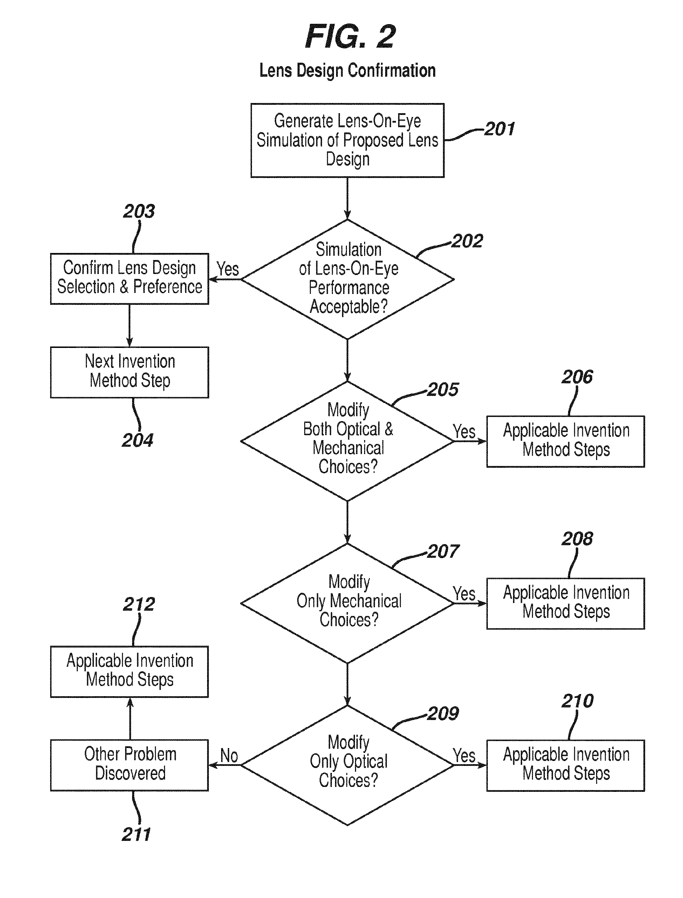

Figure 2 is a block diagram showing a system and method for providing a custom lens to a patient according to the invention, wherein a lens on eye simulation is employed to assist with product choice confirmation. Referring to Figure 2, a lens on eye simulation can be run to inform the patient of a proposed lens design when modification has been made to options previously selected by the patient 201. The patient and the practitioner determine whether the performance of the proposed lens is acceptable 202. If the patient and the practitioner deem the performance of the proposed lens acceptable, the patient and the practitioner confirm the lens design, select preferences 203 and continue with the process for manufacture of custom lenses 204. If the patient and the practitioner do not deem the performance of the proposed lens to be acceptable, optical and mechanical choices can be modified 205, before the patient and the practitioner select preferences and continue with the process for manufacture of custom lenses 206. If the patient and the practitioner deem the optical choices acceptable but do not deem the mechanical choices to be acceptable, the mechanical choices can be modified 207 before the patient and the practitioner select preferences and continue with the process for manufacture of custom lenses 208. If the patient and the practitioner deem the mechanical choices acceptable but do not deem the optical choices to be acceptable, the optical choices can be modified 209 before the patient and the practitioner select preferences and continue with the process for manufacture of custom lenses 210. If neither optical nor mechanical choices are modified, but another problem discovered 211, the patient and the practitioner may need to go through the applicable steps for manufacture of custom lenses 212.

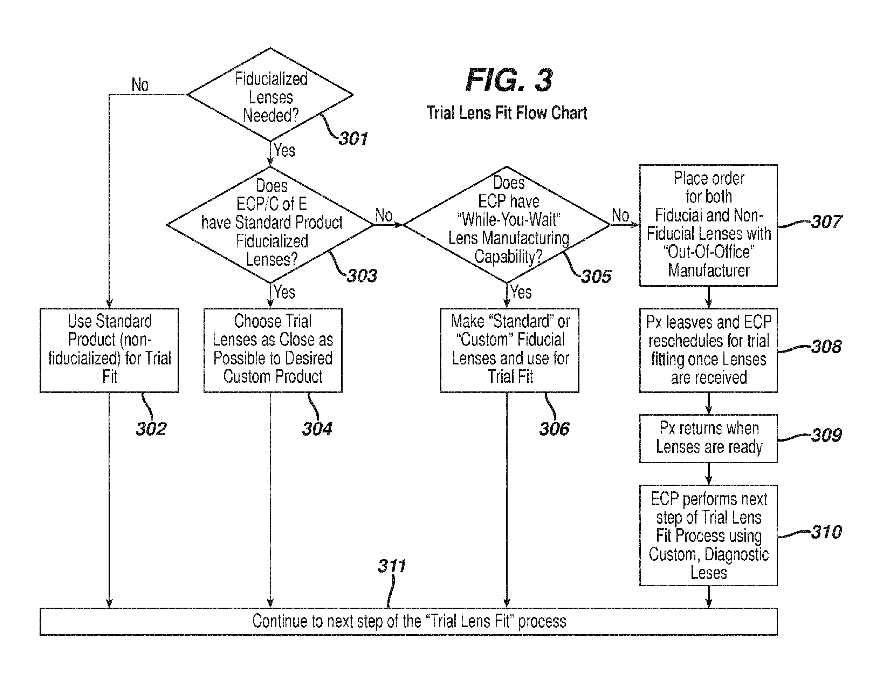

Figure 3 is a block diagram showing a system and method for providing a custom lens to a patient according to the invention, wherein a trial lens fit is employed to assist with product choice confirmation. Referring to Figure 3, whether a fiducialized lens, a non-fiducialized lens or a standard lens is needed is determined 301. If a standard product can be used for a trial fit 302, the trial lens fit process can be initiated using a standard lens 311. If a fiducialized lens is required, whether the eye care practitioner has fiducialized lenses is determined 303. If yes, trial lenses as close to desired product are chosen 304 and the trial lens fit process can be initiated 311. If no, whether the eye care practitioner has "while-you-wait" lens manufacturing capability is determined 305. If yes, a standard or custom fiducial lens is made and used for trial fit 306 and the trial lens fit process can be initiated 311. If no, the eye care practitioner places an order for fiducial and non-fiducial lenses with an "out-of-office" manufacturer 307. The patient will need to schedule another visit to have trial fit 308. The patient will return when trial lens is ready 309. The eye care practitioner performs the next step of trial lens fit process using custom diagnostic lenses 310 and the trial lens fit process can be initiated 311. In cases where trial lenses are not acceptable, a patient and eye care practitioner may go back to a product choice user readable interface. Finally, in cases where a trial lens is acceptable, centration, rotation, and movement data may be taken from the trial fit and may be either temporarily or permanently stored.

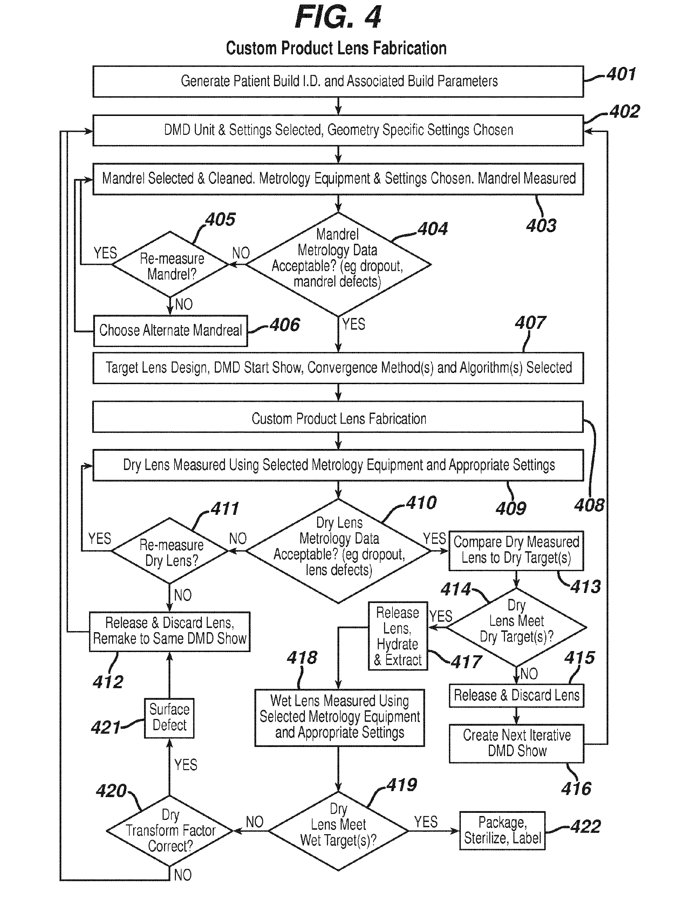

Figure 4 is a block diagram showing a system and method for providing a custom lens to a patient according to the invention, wherein custom lens manufacture is shown. Referring to Figure 4, patient build identification and associated build parameters, including target lens design, DMD start show and information stored in database, are generated 401. DMD geometry specific settings (e.g., spatial coefficients, gain, global coefficients, starting shapes, etc.) are chosen 402. A mandrel is selected and properties of the mandrel are measured using selected metrology equipment (e.g., Keyence™, Phaseview™, etc.) 403. Whether the mandrel metrology data is acceptable is determined 404. If not, the mandrel is re-measured 405 until data is acceptable and stored 403. At 404, in cases where metrology data may not be acceptable (e.g., mandrel defect), at 406, an alternate mandrel may be chosen and measured until data is acceptable. Data can then be stored. If the mandrel metrology data is acceptable, the mandrel is placed in a free form custom lens making machine; target lens design, DMD start show, convergence methods and algorithm are selected and stored 407; and a custom lens is fabricated 408. The resultant dry lens is measured using selected metrology equipment and appropriate settings 409. Whether the dry lens metrology data is acceptable is determined 410. If the dry lens metrology data is not acceptable, the dry lens is remeasured 411. If the dry lens is not acceptable, the dry lens is released and discarded and the same DMD show is remade 412. If the dry lens metrology data is acceptable, the dry lens is compared to dry targets 413 to determine if the dry lens meets the dry target 414. If the dry lens meets the dry target, the dry lens is released, hydrated and extracted 417; the resultant wet lens is measured using selected metrology equipment and appropriate settings 418; and whether the resultant wet lens meets the wet target is determined 419. If the wet lens does not meet the wet target, whether the dry transform factor is correct is determined 420. If the dry transform factor is not correct, the wet lens is released and discarded and the lens is remade 402. If the dry transform factor is correct, there may be a surface defect 421. In such a case, the lens is released and discarded and the and the lens is remade 412/402. If the wet lens meets the wet target, it is packaged, sterilized and labeled for use 422.

Figure 5 is a representative display of a simulation of vision correction for a given patient. In particular, Figure 5 is a simulated Snellen chart that is generated to show a patient the vision correction options available to him/her.

Information regarding a patient's bare eye data is provided; and two or more available vision options are selected and displayed. The patient can select based on need and preference, and can optionally purchase a custom lens. The basic steps of requesting information from a patient and selecting an appropriate option may be performed in a substantially continuous, interactive process.

For example, a store display could be equipped with an interactive computer which can prompt the user to answer questions, keep track of the answers, provide new questions and/or selections based upon the answers provided, and select an appropriate classification based on those answers as described above. Alternatively, the information may be collected from a patient though the use of an interactive site on the World Wide Web, an interactive menu-driven phone system, and the like. Charts, tables or other figures may be used as devices for requesting information from a patient and taking the patient through the preference process as described above. Similarly, charts, figures, and the like, can be distributed via e-mail, or via a network such as the World Wide Web, and the like.

It is also possible for information regarding the selection of vision correction options in accordance with the methods of the present invention to be distributed to eye care practitioners, merchants, or other persons and/or places likely to be engaged in the recommendation, retail sale, promotion, distribution, giveaway, or trade of eye care products. The interaction described in the present application could take place between a patient, a patient's caretaker, a patient's parent, a patient's eye care practitioner, merchant, or other person engaged in the sale of eye care products. Further, selection may occur proximate to a display case containing one or more of the vision correction options available within each of any available classifications.

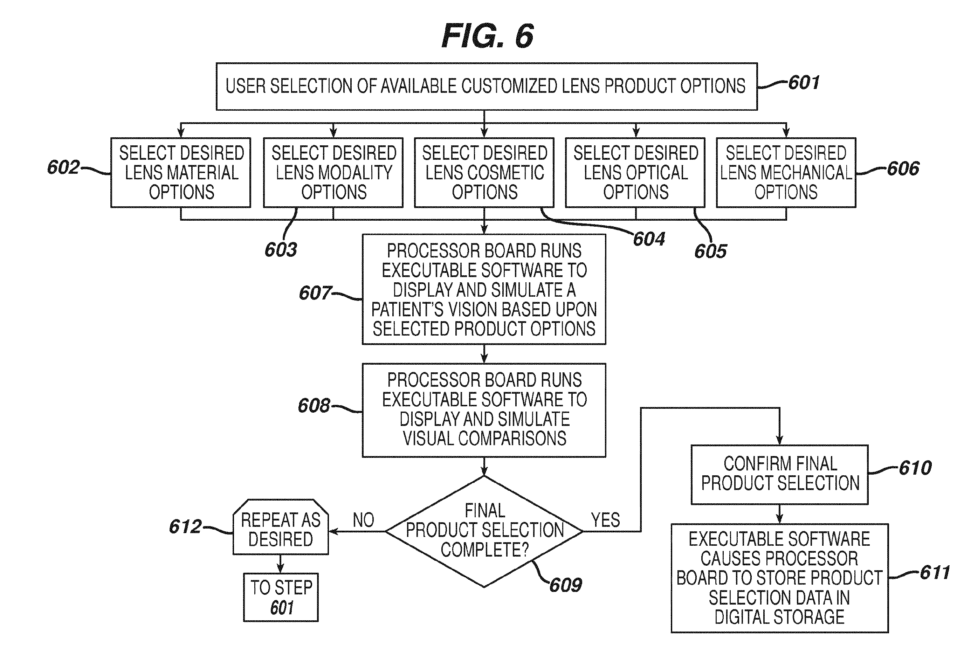

Figure 6 is a block diagram showing a system and method for providing a custom lens to a patient according to the invention. Referring to Figure 6, the patient is provided with available custom lens product options 601, based on desired lens material 602, desired lens modality 603, desired lens cosmetic features 204, desired optical features 605, and desired mechanical features 606. Processor runs executable software to display and simulate a patient's vision based upon the selected options 607, the processor runs executable software to display and simulate visual comparisons 608. The patient reviews [609] and may either ask that the simulation be repeated 612 using different parameters (if so, user selection of available custom lens product options 201 is repeated) or may make a product selection 610. If patient makes product selection, executable software causes processor to store product selection data in digital storage medium 611.

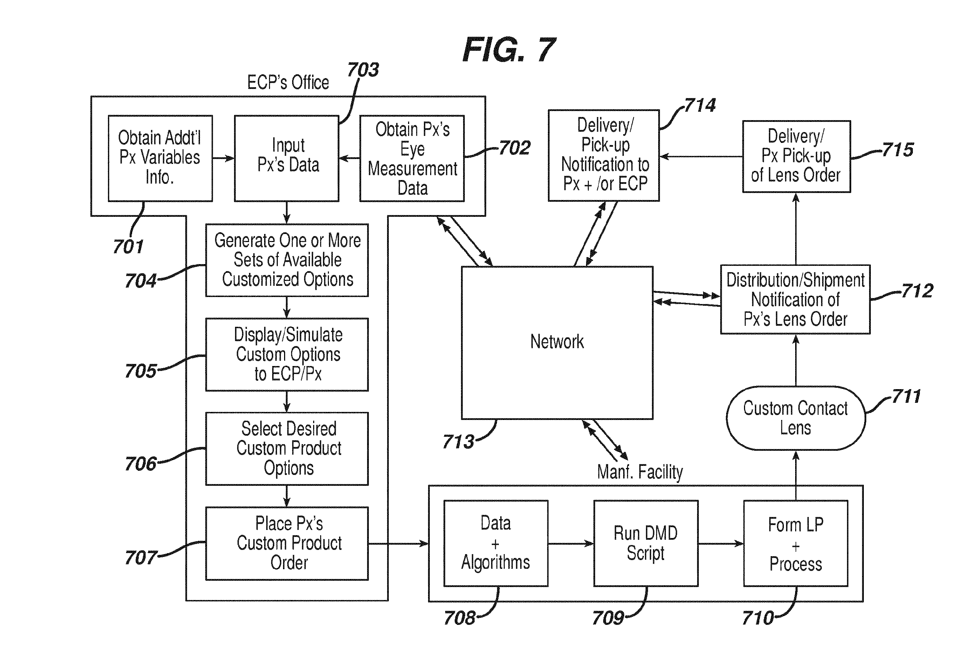

Figure 7 is a block diagram showing a system and method for providing a custom lens to a patient according to the invention. Referring to Figure 7, at the eye care practitioner's office, patient variables information 701 and patient eye measurement data 702 is collected. One or more sets of available customized options is generated 704 and a simulation of custom options is displayed 705. The patient selects the desired custom product option 706 and an order for the custom product is placed 707. At the manufacturing facility, data and algorithms regarding the order 708 are used to run DMD script 709 to form Lens Precursor to process 710 in order to prepare a custom contact lens 711. Notification of shipment of the custom contact lens 712 is provided through a network 713. Delivery/pick up notification is provided to the eye care practitioner and/or the patient 714 and the custom lens is delivered/picked up 715.

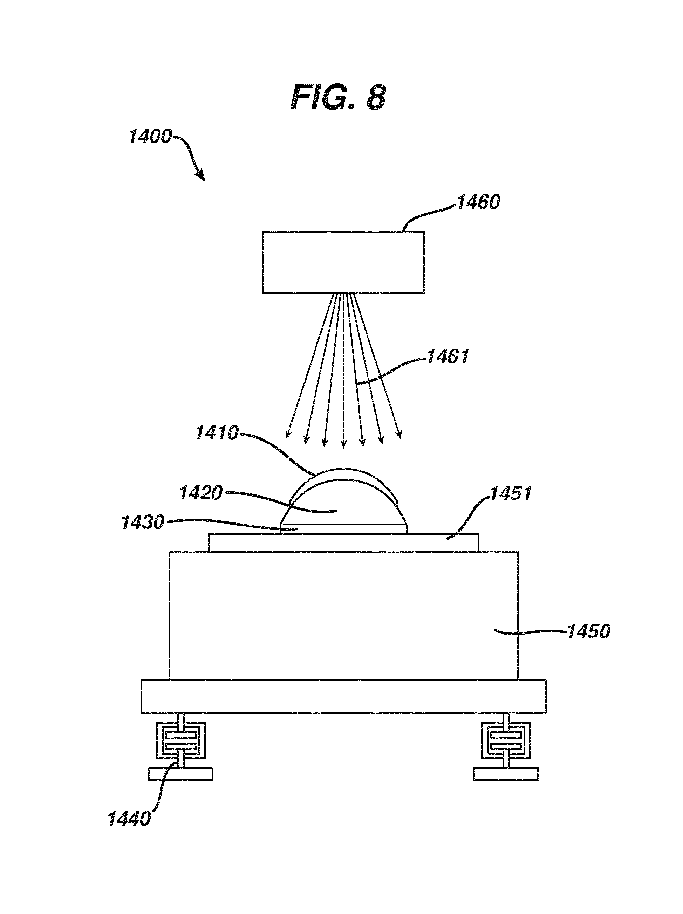

Figure 8, which was taken from U.S. Patent No. 7,905,594, shows an apparatus 1400 that may be useful in manufacturing a custom contact lens in accordance with the invention. As disclosed in U.S. Patent No. 7,905,594, apparatus includes table 1450 supported by a vibration isolation system 1440. A Lens Precursor 1410 (as this term is defined in U.S. Patent No. 7,905,594) is attached to a forming optic holder 1430 which in turn may be attached with a holding apparatus 1451. A source of fixing radiation 1460 exposes the Lens Precursor and surroundings to the fixing radiation 1461 and an ophthalmic lens is formed.
The foregoing examples are not intended to limit the scope of the present invention, which may be set out in the claims. In particular, various equivalents and substitutions will be recognized by those skilled in the art in view of the foregoing disclosure and these are contemplated to be within the scope of the invention.

A non-exhaustive list of various aspects and examples of the present invention are set out in the following numbered clauses:
Clause 1. A method for providing vision correction to a patient, comprising: a) obtaining at least one bare eye data measurement, habitual lens data measurement and/or fitting lens data measurement of the patient; b) providing said at least one bare eye data measurement, habitual lens data measurement and/or fitting lens data measurement to a free form custom lens manufacturing system; c) producing a customized lens using the free form custom lens manufacturing system; and d) providing said custom lens to the patient.
Clause 2. The method according to clause 1, wherein said customized lens is one of a contact lens, an inlay, an onlay, and an IOL.
Clause 3. The method according to clause 1, wherein said at least one bare eye data measurement, habitual lens data measurement and/or fitting lens data measurement is obtained by at least one of a wavefront sensing technique and a corneal topography technique.
Clause 4. The method according to clause 1, wherein providing said at least one bare eye data measurement, habitual lens data measurement and/or fitting lens data measurement to said free form custom lens manufacturing system comprises at least one of voice signals and data signals transmitted over a communication system.
Clause 5. The method according to clause 4, wherein said voice or data signals are transmitted over at least one of a land-based and a wireless-based communication system.
Clause 6. The method according to clause 1, wherein said at least one bare eye data measurement, habitual lens data measurement and/or fitting lens data measurement is taken at location selected from the group consisting of a practitioner's office, at a retail store, at a kiosk, at a vehicle, at a patient's workplace and at a patient's home.
Clause 7. The method according to clause 5, wherein said free form custom lens manufacturing system is located substantially proximal to said practitioner's office.
Clause 8. The method according to clause 5, wherein said free form custom lens manufacturing system is located substantially remote from said practitioner's office.
Clause 9. The method according to clause 1, further comprising obtaining a patient history data.
Clause 10. The method according to clause 1, wherein said at least one bare eye data measurement includes corneal topography information.
Clause 11. The method according to clause 1, wherein providing said custom lens comprises a personalized presentation to said patient.
Clause 12. A method for providing vision correction to a patient, comprising: a) obtaining at least one bare eye data measurement, habitual lens data measurement and/or fitting lens data measurement; and b) providing a display of available vision correction for said at least one bare eye data measurement, habitual lens data measurement and/or fitting lens data measurement in the form of at least one of a picture, a computer simulation, a graphic display.
Clause 13. The method according to clause 12, wherein providing said display comprises providing said patient with said display such that said patient may make a subjective evaluation of the available vision correction.
Clause 14. The method of clause 13, wherein an option matrix that includes at least a comparison of prospective vision correction as a function of a prospective eye treatment is provided.
Clause 15. The method of clause 14, wherein said prospective eye treatment includes at least one standard lens, at least one custom lens, and no treatment.
Clause 16. An apparatus for forming a custom lens, comprising:
   a computer processor in digital communication with a digital media storage device, wherein the digital media storage device stores software code;
   a transmitter in logical communication with the processor and also in logical communication with a communication network, wherein the software is executable upon demand and operative with the processor to:
      receive data descriptive of a characteristic of a patient's eye;
      receive data descriptive of a fitting lens measurement; and
      transmit the data descriptive of a characteristic of the patient's eye and the data descriptive of a fitting lens measurement to a manufacturing apparatus; wherein the manufacturing apparatus generates one or more custom lenses based upon the data descriptive of the characteristic of the patient's eye and the data descriptive of the fitting lens measurement.
Clause 17. The apparatus of clause 1, wherein the software is additionally operative with the processor to transmit the data descriptive of a characteristic of a patient's eye and the data descriptive of a fitting lens measurement to a manufacturing apparatus via the communication network.
Clause 18. The apparatus of clause 1, wherein the data descriptive of a characteristic of a patient's eye comprises bare eye data.
Clause 19. The apparatus of clause 1, wherein the data descriptive of a characteristic of a patient's eye comprises habitual lens data.
Clause 20. The apparatus of clause 1, wherein the software is additionally operative with the processor to generate and transmit simulation data through the communication network, wherein the simulation data generated comprises one or more measurements related to the patient's vision based upon a modeled use of the patient wearing the custom lens as compared to one or both of: the patient wearing a standard lens and the patient's vision without any vision correction lens.

## Claims

1. A method for providing vision correction to a patient, comprising: a) obtaining at least one bare eye data measurement, habitual lens data measurement and/or fitting lens data measurement of the patient; b) providing said at least one bare eye data measurement, habitual lens data measurement and/or fitting lens data measurement to a free form custom lens manufacturing system; c) producing a customized lens using the free form custom lens manufacturing system; and d) providing said custom lens to the patient.

2. The method according to claim 1, wherein said customized lens is one of a contact lens, an inlay, an onlay, and an IOL.

3. The method according to claim 1 or claim 2, wherein said at least one bare eye data measurement, habitual lens data measurement and/or fitting lens data measurement is obtained by at least one of a wavefront sensing technique and a corneal topography technique.

4. The method according to any of the preceding claims, wherein providing said at least one bare eye data measurement, habitual lens data measurement and/or fitting lens data measurement to said free form custom lens manufacturing system comprises at least one of voice signals and data signals transmitted over a communication system.

5. The method according to claim 4, wherein said voice or data signals are transmitted over at least one of a land-based and a wireless-based communication system.

6. The method according to any of the preceding claims, wherein said at least one bare eye data measurement, habitual lens data measurement and/or fitting lens data measurement is taken at location selected from the group consisting of a practitioner's office, at a retail store, at a kiosk, at a vehicle, at a patient's workplace and at a patient's home.

7. The method according to claim 6, wherein said free form custom lens manufacturing system is located substantially proximal to said practitioner's office.

8. The method according to claim 6, wherein said free form custom lens manufacturing system is located substantially remote from said practitioner's office.

9. The method according to any of the preceding claims, further comprising obtaining a patient history data.

10. The method according to any of the preceding claims, wherein said at least one bare eye data measurement includes corneal topography information.

11. The method according to any of the preceding claims, wherein providing said custom lens comprises a personalized presentation to said patient.

12. A method for providing vision correction to a patient, comprising: a) obtaining at least one bare eye data measurement, habitual lens data measurement and/or fitting lens data measurement; and b) providing a display of available vision correction for said at least one bare eye data measurement, habitual lens data measurement and/or fitting lens data measurement in the form of at least one of a picture, a computer simulation, a graphic display.

13. The method according to claim 12, wherein providing said display comprises providing said patient with said display such that said patient may make a subjective evaluation of the available vision correction.

14. The method of claim 13, wherein an option matrix that includes at least a comparison of prospective vision correction as a function of a prospective eye treatment is provided.

15. The method of claim 14, wherein said prospective eye treatment includes at least one standard lens, at least one custom lens, and no treatment.

16. An apparatus for forming a custom lens, comprising:
a computer processor in digital communication with a digital media storage device, wherein the digital media storage device stores software code;
a transmitter in logical communication with the processor and also in logical communication with a communication network, wherein the software is executable upon demand and operative with the processor to:
receive data descriptive of a characteristic of a patient's eye;
receive data descriptive of a fitting lens measurement; and
transmit the data descriptive of a characteristic of the patient's eye and the data descriptive of a fitting lens measurement to a manufacturing apparatus; wherein the manufacturing apparatus generates one or more custom lenses based upon the data descriptive of the characteristic of the patient's eye and the data descriptive of the fitting lens measurement.

17. The apparatus of claim 16, wherein the software is additionally operative with the processor to transmit the data descriptive of a characteristic of a patient's eye and the data descriptive of a fitting lens measurement to a manufacturing apparatus via the communication network.

18. The apparatus of claim 16 or claim 17, wherein the data descriptive of a characteristic of a patient's eye comprises bare eye data.

19. The apparatus of claim 16 or claim 17, wherein the data descriptive of a characteristic of a patient's eye comprises habitual lens data.

20. The apparatus of any of claims 16 to 19, wherein the software is additionally operative with the processor to generate and transmit simulation data through the communication network, wherein the simulation data generated comprises one or more measurements related to the patient's vision based upon a modeled use of the patient wearing the custom lens as compared to one or both of: the patient wearing a standard lens and the patient's vision without any vision correction lens.
